# EUROPÄISCHE PATENTSCHRIFT

(11) **EP 4 146 333 B1**
(45) Veröffentlichungstag und Bekanntmachung des Hinweises auf die Patenterteilung: **20.08.2025**
(21) Anmeldenummer: 21724600.8
(22) Anmeldetag: 04.05.2021
(51) Int. Cl.: A61N 1/375, H01R 13/52, H01R 24/58, H01R 31/06, A61M 5/142, A61N 1/05

(54) **KOPFTEIL EINER IMPLANTIERBAREN MEDIZINISCHEN VORRICHTUNG UND VERFAHREN ZUR HERSTELLUNG DES KOPFTEILS**
HEAD PART FOR AN IMPLANTABLE MEDICAL DEVICE AND METHOD FOR PRODUCING THE HEAD PART
PARTIE DE TÊTE POUR UN DISPOSITIF MÉDICAL IMPLANTABLE ET MÉTHODE DE FABRICATION DE LA PARTIE DE TÊTE

(30) Priorität: 05.05.2020 DE 102020112084
(43) Veröffentlichungstag der Anmeldung: 15.03.2023
(73) Patentinhaber: Neuroloop GmbH, 79110 Freiburg (DE)
(72) Erfinder: KIMMIG, Fabian, 79110 Freiburg (DE); BORETIUS, Tim, 79110 Freiburg (DE)
(74) Vertreter: Rösler, Uwe
(86) Internationale Anmeldenummer: PCT/EP2021/061682
(87) Internationale Veröffentlichungsnummer: WO 2021/224231

(56) Entgegenhaltungen:
- WO-A1-2019/115176
- DE-A1- 102018 124 307
- US-A1- 2003 018 364
- US-A1- 2013 245 710
- US-A1- 2014 237 806
- US-B1- 6 390 843

## Beschreibung

### Technisches Gebiet

Die Erfindung bezieht sich auf ein Kopfteil einer implantierbaren medizinischen Vorrichtung, dessen Herstellverfahren sowie eine in das Kopfteil fügbare Steckeranordnung. Das Kopfteil weist ein Kopfteilgehäuse auf, das wenigstens eine sacklochartige Steckerkontaktbuchse mit einer Buchsenöffnung sowie einen der Buchsenöffnung axial gegenüberliegenden Buchsenboden besitzt, längs der in koaxialer Anordnung und axial serieller Abfolge wenigstens ein elektrisch leitendes Kontaktringelement sowie ein elektrisch isolierender, elastisch verformbarer Dichtungsring gefügt sind, die von einer erstarrten Vergussmasse umschlossen sind.

### Stand der Technik

Implantierbare medizinische Vorrichtungen zum Zwecke der elektrischen Stimulation lokaler intrakorporaler Gewebe- oder Nervenbereiche, kurz implantierbare Impulsgeber (IPG), beispielsweise für herztherapeutische Defribillations-, Schrittmacher- sowie Resynchronisationsanwendungen, für neurostimulationstherapeutische Maßnahmen, wie bspw. Rückenmarkstimulation, Hirnstimulation oder Vagusnervstimulation, um nur einige zu nennen, weisen in aller Regel ein in sich abgeschlossenes Gehäuse auf, in dem Komponenten zur elektrischen Pulsgeneration enthalten sind, so zumindest eine elektrische Energiequelle und eine mit dieser verbundenen, elektrischen Schaltungsstruktur. Zudem schließt an das Gehäuse ein sogenanntes Kopfteil an, in dem eine mit der Energiequelle bzw. der elektrischen Schaltungsstruktur verbundene, elektrische Kontaktanordnung enthalten ist, in die eine mit dem Kopfteil fluiddicht abschließende Stecker Anordnung fügbar ist, die mit elektrischen Zu- und Ableitungen zum Zwecke einer intrakorporalen lokalen Applikation elektrischer Stimulationssignale sowie gegebenenfalls der Zuführung intrakorporal lokal abgegriffener elektrischer Signale zur Gehäuseseitig vorhandenen elektrischen Schaltungsstruktur, kontaktiert ist.

In der Druckschrift EP 2 134 418 B1 ist ein gattungsgemäßes Kopfteil einer implantierbaren medizinischen Vorrichtung beschrieben, das zwei längs einer Fügenaht zusammenfügbare Kopfteilgehäusehälften umfasst, in die jeweils in serieller Abfolge durch Zwischenwände beabstandet, halbzylinderförmige Ausnehmungen eingebracht sind, in die in jeweils seriell abwechselnder Reihenfolge elektrisch leitende Kontaktringelemente sowie elektrisch isolierende Dichtungsringe eingesetzt sind. Das Kopfteil umfasst somit eine Anordnung koaxial zueinander ausgerichteter und elektrisch isolierter Kontaktringelemente, zu deren elektrischen Kontaktierung ein seitlicher Zugang im Kopfteil vorgesehen ist, durch den eine elektrische Steckeranordnung in einen von sämtlichen ringförmigen Kontaktringelementen umfassten Hohlraum fluiddicht fügbar ist.

Die Druckschrift DE 10 2012 010 901 A1 offenbart ein Verfahren zum Positionieren und Halten von elektrischen Kontakten und Dichtungen innerhalb eines Kopfteils zur elektrischen Kontaktierung an eine medizinische implantierbare Vorrichtung. In das aus einem bioverträglichen und elektrisch isolierenden Material bestehende Kopfteilgehäuse ist einseitig eine sacklochartige Bohrung eingebracht, in der in abwechselnder Reihenfolge elektrisch leitende Kontaktringe und ringförmige Dichtelemente eingebracht sind, die gemeinsam einen Hohlraum umfassen, in den eine stiftförmige Steckeranordnung fügbar ist. Jeder der einzelnen ringförmigen Kontaktringe ist innerhalb des Kopfteils über eine elektrische Verbindungsleitung mit innerhalb des Gehäuses der medizinische implantierbare Vorrichtung befindlichen elektrischen Komponenten verbunden.

In der Druckschrift DE 20 2013 012 073 U1 ist eine Steckerbohrung-Modulbaugruppe offenbart, zu deren Montage eine Anzahl von Kontaktringen und Dichtelementen in abwechselnder Reihenfolge längs eines stiftförmigen Montagewerkzeuges angeordnet ist. Mittels einer Spannvorrichtung werden sämtliche längs des Montagewerkzeuges aufsitzenden Kontaktringe und Dichtelemente unter Aufbringung einer axialen Fügekraft gegeneinander verspannt. Zum Zwecke der Konservierung der Fügekraft dient ein mittels einer Madenschraube axialfest auf dem Montagewerkzeug aufsitzendes Hülsenelement, das zusammen mit einem endseitigen Montagewerkzeugkopf die Anordnung aus Kontaktringen und Dichtelementen axial beidseitig begrenzt. In diesem verspannten Zustand erfolgt ein Vergießen der Anordnung mit einer aushärtbaren Vergussmasse, die im erstarrten Zustand die Fügekraft aufnimmt.

Anstelle einer Madenschraube als endseitige Fixierhilfe zur Konservierung der auf die längs des stiftförmigen Montagewerkzeuges angeordneten Reihenfolge aus Kontaktring- und Dichtringelementen wirkenden axialen Fügekraft ist in der Druckschrift WO 2019/115176 A1 vorgeschlagen, eine mit einer Öffnung und Innengewinde versehene Montageplatte einzusetzen, in deren Innengewindeöffnung das stiftförmige Montagewerkzeug mit einem an dessen Ende angeordnetes Außengewinde gefügt ist.

Der Druckschrift US 2008/0077190 A1 ist ein Kopfteil zu entnehmen, das über zwei parallel und lateral versetzt zueinander verlaufende Steckerkontaktbuchsen zum Einfügen jeweils eines koaxialen Steckerteils verfügt. Koaxial zu den Buchsen mündet jeweils endseitig am Buchsenboden ein stirnseitiges Ende einer Glasfaseranordnung, die an ihrem anderen Faserende an einem optischen Lichtpulsgenerator ankoppelt.

Die Druckschrift US 2011/0004279 A1 beschreibt ein Kopfteil, über das zwei elektrische Steckerteile durch koaxiales Einfügen in einen gemeinsamen Buchsenkanal durch jeweils sich zwei axial gegenüberliegende Buchsenkanalöffnungen miteinander elektrisch kontaktierbar sind.

Der Druckschrift DE 10 218 124 307 A1 offenbart einen Stecker für ein medizinisches Implantat sowie dessen Herstellverfahren, mit einer Vielzahl an seiner zylindrischen Aussenseite, jeweils elektrisch isoliert voneinander getrennt, angeordneten Kontaktringelementen. Zudem umschließt der Stecker einen den Stecker in Längsrichtung durchsetzendes Innenlumen, das von einem Medium durchströmbar ist.

Die Druckschrift US 2013/245710 A1 erläutert ein Kopfteil für einen Herzschrittmacher, das wenigstens ein Lumen zur Aufnahme einer Zuführleitung bzw. eines Stiletts aufweist.

Die Druckschrift US 2014/237806 A1 offenbart ein Verfahren zur Herstellung eines Kopfteils eines medizinischen Implantats mit einer Ball-Seed-Verbindungsstrutur.

Die Druckschrift US 2003/0018364 A1 offenbart eine implantierbare Steckeranordnung am Kopfteil eines elektrischen Implantats, die fluiddicht gegenüber dem intrakorporalen Milieu ausgebildet ist. Die Druckschrift US 6 390 843 B1 offenbart eine entsprechende implantierbare Steckeranordnung am Kopfteil eines elektrischen Implantats.

### Darstellung der Erfindung

Der Erfindung liegt die Aufgabe zugrunde ein Kopfteil einer implantierbaren medizinischen Vorrichtung, dessen Herstellverfahren sowie eine in das Kopfteil fügbare Steckeranordnung derart zu modifizieren, so dass es neben der bisher bekannten Erzeugung, Übertragung und Applikation von elektrischen Stimulationssignalen möglich sein soll den Funktionsumfang von implantierbaren Impulsgebern (IPG) zu vergrößern ohne dabei deren Baugröße nennenswert zu ändern.

Die Lösung der der Erfindung zugrunde liegenden Aufgabe ist im Anspruch 1 angegeben. Im Anspruch 15 ist ein Verfahren zur Herstellung des Kopfteils angegeben. Den Erfindungsgedanken in vorteilhafter Weise weiterbildende Merkmale sind Gegenstand der Unteransprüche sowie der Beschreibung, insbesondere unter Bezugnahme auf die illustrierten Ausführungsbeispiele zu entnehmen.

Das lösungsgemäße Kopfteil einer implantierbaren medizinischen Vorrichtung, mit einem Kopfteilgehäuse, das wenigstens eine erste sacklochartige Steckerkontaktbuchse mit einer Buchsenöffnung sowie einem der Buchsenöffnung axial gegenüberliegenden Buchsenboden besitzt, längs der in koaxialer Anordnung und axial serieller Abfolge wenigstens ein elektrisch leitendes Kontaktringelement sowie ein elektrisch isolierender, elastisch verformbarer Dichtungsring gefügt sind, die von einer erstarrten Vergussmasse umschlossen sind, zeichnet sich dadurch aus, dass wenigstens eine zweite sacklochartige Steckerkontaktbuchse mit einer Buchsenöffnung sowie einem der Buchsenöffnung axial gegenüberliegenden Buchsenboden innerhalb des Kopfteils angeordnet ist, längs der in koaxialer Anordnung und axial serieller Abfolge wenigstens ein elektrisch leitendes Kontaktringelement sowie ein elektrisch isolierender, elastisch verformbarer Dichtungsring gefügt sind, die von der erstarrten Vergussmasse umschlossen sind, dass die Buchsenböden der wenigstens zwei Steckerkontaktbuchsen jeweils von einem von der erstarrten Vergussmasse zumindest teilweise umschlossenen Verbindungsmittel begrenzt sind, und dass das Verbindungsmittel einen Durchgangskanal einschliesst, der beidseitig in die sacklochartigen Steckerkontaktbuchsen offen mündet.

Die der Erfindung zugrunde liegende Idee macht sich die mit dem Innengewinde versehene Öffnung innerhalb der Montageplatte, die in der vorstehend erläuterten Druckschrift WO 2019/115176 A1 beschrieben ist und dort nach Entnahme des Montagewerkzeuges aus dem fertiggestellten Kopfteil ansonsten funktionslos bleibt, zunutze, um durch die Öffnung hindurch einen Medienstrom beliebiger Art zu richten. Die im vorgenannten Stand der Technik als eine Art verlorenes Bauelement anzusehende Montageplatte, die vollständig von der Vergussmasse des Kopfteils umhüllt ist und innerhalb der erstarrten Vergussmasse für den weiteren Betrieb des Kopfteils sowie auch des mit dem Kopfteil verbundenen implantierbaren Impulsgeber (IPG) ansonsten funktionslos ist, bildet die zentrale Komponente des modifizierten, lösungsgemäßen Kopfteils, die die zusätzliche Funktion eines Verbindungskanals erhält, durch den ein Medienstrom von einer sacklochartigen Steckerkontaktbuchse zur anderen sacklochartigen Steckerkontaktbuchse gerichtet werden kann. Dies eröffnet eine Vielzahl neuartiger Anwendungen für das an sich bekannte Implantat, dessen Hauptfunktion, die intrakorporale elektrische Stimulation lokaler Gewebe- und/oder Nervenbereiche, davon unbeeinträchtigt bleibt.

Das mittel- oder unmittelbar die Buchsenböden der wenigstens zwei Steckerkontaktbuchsen begrenzende Verbindungsmittel dient in lösungsgemäßer Weise zusätzlich als Medienkopplungsstück, über das bzw. durch das ein Medium vorgegebener Wahl von einer Steckerkontaktbuchse in den Bereich der anderen Steckerkontaktbuchse überführbar ist. In beiden Steckerkontaktbuchsen ist jeweils eine lösungsgemäß modifizierte Steckeranordnung lösbarfest fügbar, die jeweils zum Fügen in einer der sacklochartigen Steckerkontaktbuchsen des vorstehend bezeichneten Kopfteils entsprechend ausgebildet und angepasst sind. Insbesondere verfügen die Steckeranordnungen über einen Steckerkorpus mit wenigstens einem innenliegenden Hohlkanal, der jeweils einseitig am distalen Steckerkorpusende mündet. Überdies weisen die distalen Steckerkorpusenden eine Fügekontur auf, die im gefügten Zustand innerhalb der Steckerkontaktbuchse einen bündigen Übergang des steckerseitigen Hohlkanals zu dem innerhalb des Verbindungsmittels angeordneten und jeweils in die sacklochartige Steckerkontaktbuchse offen mündenden Durchgangskanal gewährleistet. Hierdurch soll eine möglichst verlustfreie Ankopplung und Weiterleitung bzw. Übertragung eines innerhalb der Hohlkanäle bzw. des Durchgangskanals geführten Mediums zwischen beiden Steckeranordnungen über das Verbindungsmittel realisierbar sein.

Die implantierbaren Steckeranordnungen sind ansonsten in an sich bekannter Weise zur Übertragung elektrischer Signale ausgebildet und weisen in Steckerlängserstreckung eine serielle Abfolge von Steckerkontaktringen sowie Dichtungsringen bzw. elektrische Isolationsringe auf, entsprechend der Anzahl und Anordnung der elektrischen Kontaktringelemente und Dichtungsringen längs der Steckerkontaktbuchsen.

Im gefügten Zustand der implantierbaren Steckeranordnungen innerhalb der kopfteilseitig vorgesehenen Steckerkontaktbuchsen bleibt die elektrische Funktionalität der implantierbaren Vorrichtung zur Applikation elektrischer Stimulationssignale trotz modifiziertem Kopfteil sowie modifizierter Steckeranordnungen uneingeschränkt erhalten. Über die jeweils zusätzlich steckerseitig als Medienkanäle vorgesehenen und ausgebildeten Hohlkanäle, die über den Durchgangskanal innerhalb des Verbindungsmittels miteinander verbindbar sind, lässt sich ein intrakorporaler Medientransport und/oder eine intrakorporale Medienapplikation in Abhängigkeit eines jeweils gewählten Mediums, bspw. in Form eines Gas- oder Flüssigkeitstransportes oder -applikation oder in Form einer Lichtapplikation realisieren.

Eine erste Ausführungsform sieht die Ausbildung der Hohlkanäle sowie des Durchgangskanals jeweils als Fluidleitung vor, längs der ein gasförmiges oder flüssiges Medium hindurchgeleitet bzw. transportiert werden kann.

Unmittelbar an den aus dem Kopfteil herausragenden Steckeranordnungen schließt jeweils eine abführende, vorzugsweise flexibel ausgebildete Leitung an, längs der sowohl wenigstens eine elektrische Leitung, über die elektrische Stimulationssignale bzw. körpereigene, sensorisch erfasste elektrische Signale übertragbar sind, als auch der wenigstens eine Hohlkanal zur Medienübertragung verläuft.

Je nach intrakorporaler Anordnung und Verlegung der von den Steckeranordnungen abführenden Leitungen lassen sich unterschiedliche intrakorporale Bereiche über die mit beiden Steckeranordnungen verbundenen Hohlleitungen und den die beiden Hohlleitungen miteinander verbindenden Durchgangskanal fluidisch miteinander koppeln.

Die fluidische Kopplung kann in einer einfachsten Variante rein passiv erfolgen, d. h. ohne Zwischenschaltung einer fluidischen Fördereinheit.

Eine bevorzugte Ausführungsform sieht innerhalb des Verbindungsmittels längs des Durchgangskanals eine Fluidpumpe vor, zu deren Ansteuerung die Fluidpumpe mit elektrischen Komponenten verbunden ist, die innerhalb der implantierbaren Vorrichtung zur elektrischen Pulserzeugung angeordnet sind. Die kontrolliert ansteuerbare Fluidpumpe ermöglicht somit einen intrakorporalen Fluidstrom, der Fluid aus einem Körperbereich, in dem die mit einer der beiden Steckeranordnungen verbundene Hohlleitung offen mündet, in einen anderen Körperbereich überführt, in dem die mit der anderen Steckeranordnung verbundene Hohlleitung offen mündet. Die Aktivierung der Fluidpumpe sowie auch die Vorgabe von Strömungsrichtung und Strömungsstärke sind mit Hilfe der in der implantierbaren Vorrichtung enthaltenen elektrischen Komponenten in geeigneter Weise vorgebbar.

In einer weiteren Ausführungsform umfasst die implantierbare Vorrichtung ein Fluidreservoir, das über eine Verbindungsleitung fluidisch mit dem Durchgangskanal innerhalb des Verbindungsmittels ankoppelbar ist, so dass auf diese Weise, vorzugsweise über eine längs der Verbindungsleitung ansteuerbare Ventileinheit ein zusätzliches Fluid, vorzugsweise in Form eines gasförmigen oder flüssigen Wirkstoffes in die durch den Durchgangskanal hindurchtretende Fluidströmung zumischbar ist.

Alternativ oder in Kombination zur Ausbildung der Hohlleitungen sowie des Durchgangskanals als Fluidleitung sieht eine weitere Ausführungsform das Vorsehen eines lichtleitenden Mediums längs der Hohlkanäle sowie des Durchgangskanals vor. Auf diese Weise lassen sich zwei räumlich getrennt voneinander angeordnete intrakorporale Bereiche optisch miteinander koppeln. Hierzu sind jeweils längs der mit den Steckeranordnungen verbundenen Hohlleitungen jeweils wenigstens ein Lichtleiter verlegt, deren jeweils an den Steckerkorpusenden der Steckeranordnung offen mündenden Lichtleiterenden im gefügten Zustand innerhalb des Kopfteils möglichst verlustfrei an einen innerhalb des Durchgangskanals angeordneten Lichtleiter ankoppeln. Vorzugsweise ist längs des Durchgangskanals innerhalb des Verbindungsmittels zusätzlich eine optische Einheit angeordnet, die Einfluss auf das längs die einzelnen Lichtleiter übertragene Licht zu nehmen vermag. Bspw. ist längs des Durchgangskanals ein optisches Ein- und/oder Auskoppelelement vorgesehen, über das Licht einer innerhalb der implantierbaren Vorrichtung vorgesehenen Lichtquelle längs des Lichtleiters einkoppelbar und/oder über das Licht zur Übertragung eines innerhalb der implantierbaren Vorrichtung vorgesehenen Lichtdetektors aus dem Lichtleiter auskoppelbar ist.

Alternativ oder in Ergänzung zum Vorsehen eines optischen Ein- und/oder Auskoppelelementes kann ein optischer Verstärker, ein optischer Filter oder Schalter längs des Durchgangskanals innerhalb des Verbindungsmittels angeordnet sein, die mit entsprechenden innerhalb der implantierbaren Vorrichtung angeordneten elektrischen Ansteuerkomponenten verbunden sind.

Ebenso eignet es sich alternativ zu oder in Kombination mit den vorstehend genannten Komponenten zur Medienübertragung längs der Hohlkanäle sowie des Durchgangskanals ein elektrisch leitendes Medium vorzusehen, bspw. in Form wenigstens einer elektrischen Verbindungsleitung, längs der zusätzlich zu den ohnehin mit den elektrischen Kontaktringelementen verbundenen elektrischen Leitungen zum Zwecke der Gewebe- und/oder Nervenstimulation, eine zusätzliche Übertragung elektrischer Signale zwischen zwei räumlich getrennten Körperbereichen möglich wird. In diesem Fall bietet es sich in vorteilhafter Weise an, in dem Verbindungsmittel längs des Durchgangskanals eine elektrische Komponente, wie bspw. einen einstellbaren elektrischen Widerstand, einen elektrischen Verstärker oder vergleichbare elektrische Komponenten anzuordnen, um auf diese Weise den Stromfluss längs der Hohlkanäle zu beeinflussen.

In einer weiteren bevorzugten Ausführungsform ist innerhalb des Kopfteils insbesondere am Verbindungsmittel oder innerhalb des Verbindungsmittels wenigstens ein Sensor angeordnet, der das jeweils innerhalb des Durchgangskanals geführte Medium quantitativ in Bezug auf Fördervolumen, Förderrate oder Lichtintensität etc. zu detektieren bzw. sensorisch zu erfassen vermag. Je nach Art und Ausführung des wenigstens einen Sensors ist dessen Anordnung innerhalb des Kopfteils geeignet vorzunehmen, bspw. bietet es sich an, den wenigstens einen Sensor mit wenigstens einem der innerhalb des Kopfteils angebrachten elektrisch leitenden Kontaktringelementen elektrisch und/oder mechanisch direkt oder indirekt zu koppeln. Als mögliche Sensoren kommen Sensoren aus der nachfolgenden Gruppe in Frage: Ultraschallsensor, optischer Sensor, Drucksensor, Hallsensor, Flusssensor, Nadelsensor, Fluoreszenzsensor, Drucksensor, etc..

Der wenigstens eine Sensor ist vorzugsweise mit einer elektrischen Energiequelle verbunden, die zur elektrischen Energieversorgung des Pulsgenerators innerhalb der medizinischen Vorrichtung dient. Zudem ist der wenigstens eine Sensor mit einer Auswerte- und Steuereinheit verbunden, die mit einer zusätzlichen Speichereinheit und/oder mit einer innerhalb des Verbindungsmittels längs des Durchgangskanals angeordneten Komponente zur Beeinflussung der durch den Durchgangskanal hindurchtretenden Medienströmung ausgebildet ist. Auf diese Weise ist es möglich, einen sensorisch erfassten Istzustand des längs des Durchgangskanals strömenden oder geleiteten Mediums zu erfassen und die wenigsten eine im Verbindungsmittel längs des Durchgangskanals angeordnete Komponente zur Beeinflussung des Medienstromes in Abhängigkeit eines Soll-Ist-Vergleiches anzusteuern bzw. zu regeln.

Das lösungsgemäße konfektionierte Kopfteil sowie die funktionell modifizierten Steckeranordnungen zur Übertragung eines Mediums in Form eines Fluids, Lichtstroms oder eines elektrischen Stroms belässt die Form und Größe bekannter gattungsgemäßer medizinischer Implantate weitgehend unverändert, erweitert jedoch signifikant deren Funktionalität und die damit verbundene therapeutische Wirkweise vergleichbarer Implantate. So ist es möglich, neben der elektrischen Stimulation intrakorporaler lokaler Gewebe- und/oder Nervenbereiche zusätzlich ein Fluid, Licht oder elektrische Spannung zu applizieren.

Die kombinierte Nutzung des originär als Montageplatte dienenden Verbindungsmittels als Tragstruktur und als eine Art Verbindungsflansch ermöglicht eine neuartige Herstellungsweise für ein derartiges Kopfteil. So dient das Verbindungsmittel in einem ersten Verfahrensschritt als Trägerplatte im herkömmlichen Sinn, d. h. das Verbindungsmittel wird zusammen mit dem wenigstens einen Kontaktringelement sowie dem wenigstens einen Dichtungsring längs eines ersten stabförmigen Montagewerkzeuges angeordnet und dient zur Erzeugung einer axialen Spannkraft als mechanisches Gegenlager. Hierzu sieht das Verbindungsmittel eine erste Öffnung mit Innengewinde vor, in das ein am Montagewerkzeug endseitig angebrachtes Außengewinde fügbar ist. Durch Verdrehen des Montagewerkzeuges relativ zum Verbindungsmittel werden die längs des Montagewerkzeuges aufsitzenden Kontaktringelemente sowie Dichtungsringe axial gegeneinander verspannt. In gleicher Weise dient das Verbindungsmittel als Tragstruktur und mechanisches Gegenlager zur Spannkrafterzeugung längs der auf einem zweiten Montagewerkzeug angeordneten Kontaktringelementen und Dichtungsringen. Hierzu sieht das Verbindungsmittel eine zweite Öffnung mit Innengewinde vor, die mit der ersten Öffnung über den Durchgangskanal verbunden ist.

In einem nächsten Schritt wird das Verbindungsmittel mit den zwei daran befestigen Montagewerkzeugen samt der darauf aufsitzenden axialen Abfolge aus jeweils dem wenigstens einen Kontaktringelements sowie dem wenigstens einen Dichtungsringelement mit einer erstarrungsfähigen, in fließfähiger Form vorliegenden Vergussmasse zur Ausbildung des Kopfteils ummantelt. Nach Erstarren der Vergussmasse werden die stabförmigen Montagewerkzeuge aus dem Verbindungsmittel gelöst und entfernt. Durch Entfernen der Montagewerkzeuge mündet der das Verbindungsmittel durchsetzende Durchgangskanal beidseitig offen in die sich durch Entfernen der Montagewerkzeuge ausbildenden sacklochartigen Steckerkontaktbuchsen des Kopfteils.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: Darstellung eines Kopfteils mit implantierbarer Vorrichtung zur elektrischen Pulserzeugung als Konnektorvariante,
- Fig. 2: Darstellung eines Kopfteils mit implantierbarer Vorrichtung zur Erzeugung elektrischer Stimulationssignale mit innerhalb des Verbindungsmittels enthaltener, den Medienstrom beeinflussenden Komponente.

### Wege zur Ausführung der Erfindung, gewerbliche Verwendbarkeit

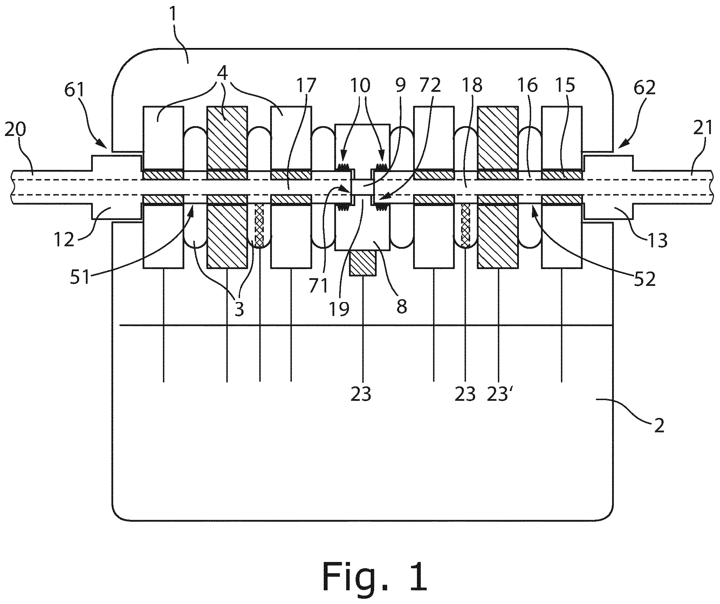

Figur 1 zeigt eine schematische Darstellung eines Kopfteils 1, das mit einer als Pulsgenerator ausgebildeten implantierbaren, medizinischen Vorrichtung 2 zu einer Baueinheit kombiniert ist. In dem aus einer erstarrungsfähigen Vergussmasse gefertigten Kopfteil sind in axial serieller Anordnung jeweils eine Vielzahl von elektrisch isolierenden, elastisch verformbaren Dichtungsringen 3 sowie elektrisch leitende Kontaktringelemente 4 in jeweils abwechselnder Reihenfolge angeordnet. Die Dichtungsringe 3 sowie die elektrischen Kontaktringelemente 4 umfassen jeweils eine sacklochartige Steckerkontaktbuchse 51, 52, die eine an der Außenwand des Kopfteils 1 zugängliche Buchsenöffnung 61, 62 besitzen. Die den Buchsenöffnungen 61, 62 gegenüberliegende Buchsenböden 71, 72 sind von einem gemeinsamen Verbindungsmittel 8 begrenzt, das für die Fertigung des Kopfteils 1 und insbesondere für eine Fügekraft beaufschlagte, axiale Aneinanderreihung der Vielzahl an Dichtringen 3 sowie elektrischen Kontaktelementen 4 als mechanisches Gegenlager für zwei jeweils zu Montagezwecken benutzte Montagewerkzeuge dient. Hierzu sieht das Verbindungsmittel 8 einen das Verbindungsmittel 8 vollständig durchsetzenden Durchgangskanal 9 vor, längs dem zumindest bereichsweise an seinen Durchgangskanalöffnungen ein Innengewinde 10 eingebracht ist.

In den sacklochartigen Steckerkontaktbuchsen 51, 52 sind jeweils eine lösungsgemäß konfektionierte Steckeranordnung 12, 13 gefügt, die jeweils einen an die Dimensionen und Formgebung der Steckerkontaktbuchsen 51, 52 angepassten Steckerkorpus aufweisen, an dessen Außenumfang entsprechend der Anordnung der elektrischen Kontaktringelemente 4, entsprechende Gegenkontaktelemente 15 angeordnet sind. Zwischen den Gegenkontaktelementen 15 sind elektrisch isolierende Steckerkorpusbereiche 16 vorgesehen. Betreffend die elektrische Kontaktierung der Steckeranordnungen 12, 13 innerhalb der jeweils sacklochartig ausgebildeten Steckerkontaktbuchsen 51, 52 des Kopfteils unterscheidet sich die in Figur 1 dargestellte Vorrichtung nicht vom vergleichbaren, gattungsgemäßen Steckeranordnungen.

In lösungsgemäßer Weise sehen die Steckerkontaktanordnungen 12, 13 jeweils einen den Steckerkorpus mittig durchsetzenden Medienkanal in Form einer Hohlleitung 17, 18 vor, der bündig im Bereich des Buchsenbodens 71, 72 an den jeweils offen in die sacklochartigen Steckerkontaktbuchsen 51, 52 mündenden Durchgangskanal 9 in Form einer Stoßverbindung angrenzt. Je nach Art und Ausbildung des Hohlkanals 17, 18 sowie auch des Durchgangskanals 9 gilt es die Ankopplung zwischen dem Durchgangskanal 9 und dem jeweils steckerseitigen Hohlkanal 17, 18 für eine verlustarme bzw. verlustfreie Ankopplung und Übertragung des durch die Hohlkanäle hindurchtretenden Mediums entsprechend auszugestalten und vorzunehmen. Optional ist hierzu innerhalb des Verbindungsmittels 8 eine zusätzliche Dichtungs- und/oder Fügekontur 19 eingebracht die für einen nahtlosen Übergang zwischen den Hohlkanälen 17, 18 und dem Durchgangskanal 9 sorgt.

Die Steckeranordnungen 12, 13 sind jeweils mit einer abführenden Leitung 20, 21 verbunden, die neben der wenigstens einen elektrischen Leitung zur elektrischen Stimulationssignalübertragung, nicht dargestellt, den Hohlkanal 17, 18 umfasst.

Das in Figur 1 illustrierte Kopfteil 1 dient in erster Linie als Konnektor bzw. Verbindungsteil zwischen zwei Steckeranordnungen 12, 13, deren zugehörige

Hohlkanäle 17, 18 über dem Durchgangskanal 19 bündig und für einen möglichen Medienübertrag verlustfrei aneinander ankoppeln.

Das in Figur 1 dargestellte Ausführungsbeispiel zeigt zwei parallel und koaxial orientierte Steckerkontaktbuchsen 51, 52, deren zugehörige Öffnungen 61, 62 an diametral gegenüberliegenden Gehäuseseiten des Kopfteils 1 münden. Gleichsam ist es möglich, zwei Steckerkontaktbuchsen in parallel nebeneinander orientierter Anordnung innerhalb des Kopfteils vorzusehen, deren zugehörige Öffnungen an ein und dergleichen Seite des Kopfteils 1 münden. In diesem Fall ist der innerhalb des Verbindungsmittels 8 geführte Durchgangskanal 9 halbbogenförmig ausgebildet, um die an den jeweiligen Buchsenböden offen mündenden steckerseitigen Medienkanäle bündig miteinander zu verbinden.

Auch ist es denkbar drei oder mehr Steckerkontaktbuchsen im Kopfteil vorzusehen, die jeweils mit einem drei- oder mehrfach abzweigenden Durchgangskanal miteinander verbunden sind.

Zur Detektion der durch die Hohlkanäle 17, 18 hindurchtretenden Medien, bspw. in Form eines gasförmigen oder flüssigen Fluids, von Licht oder elektrischen Stroms, sieht eine bevorzugte Ausführungsvariante das Vorsehen wenigstens eines Sensors 23 innerhalb des Kopfteils 1 vor. Je nach Art und Ausbildung des Sensors 23 ist dieser unmittelbar am Verbindungsmittel 8 und/oder an wenigsten einem der Dichtungsringe 3 oder wenigstens einen der elektrischen Kontaktringelemente 4 angeordnet oder in diesen integriert. Die Art und Anbringung des wenigstens einen Sensors 23 richtet sich nach dem längs der Hohlkanäle 17, 18 übertragenen Medium. Als Sensor 23 kann in geeigneter Weise aus den nachfolgenden Sensortypen gewählt werden: Hallsensor, Optiksensor, Flusssenor, Ultraschallsensor, Temperatursensor, Drucksensor etc..

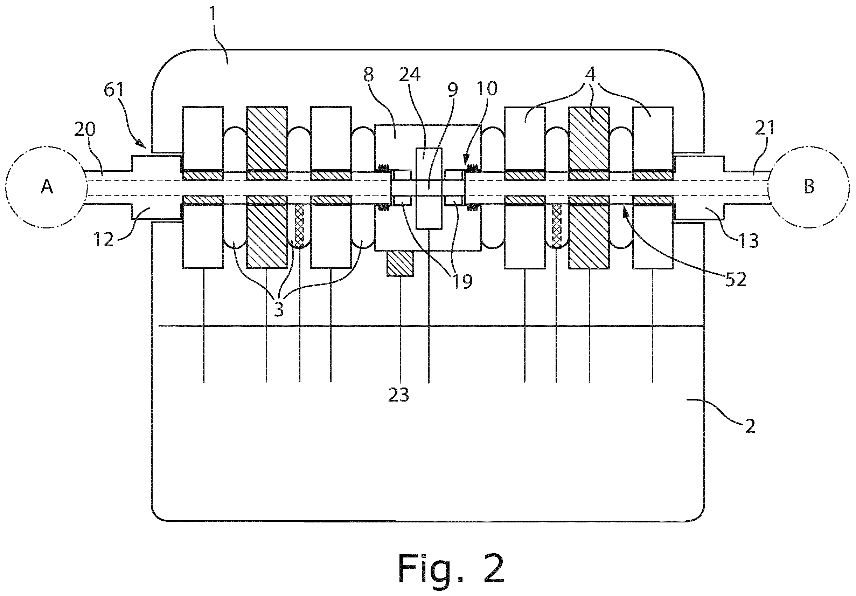

In Figur 2 ist im Unterschied zu dem in Figur 1 illustrierten Kopfteils 1 innerhalb des Verbindungsmittels 8 eine zusätzliche Komponente 24 längs des Durchgangskanals 9 angeordnet, die Einfluss auf das Medium hat, das durch die Hohlkanäle 17, 18 sowie den Durchgangskanal 9 hindurchtritt. In Figur 2 sind bau- und funktionsgleiche Komponenten, die bereits in Zusammenhang mit dem in Figur 1 gezeigten Ausführungsbeispiel 1 beschrieben worden sind, mit gleichen Bezugszeichen versehen.

Im Falle von als Fluidleitungen ausgebildete Hohlkanäle 17, 18 stellt die längs des Durchgangskanals 9 angeordnete Komponente 24 vorzugsweise eine Fluidpumpe dar. Mit Hilfe der Fluidpumpe ist es möglich bspw. intrakorporale Flüssigkeit aus einem Körperbereich A in einen andern Körperbereich B zu fördern. In Ergänzung kann die Komponente 24 einen Filter zur Aufreinigung des durch den Durchgangskanal 9 hindurchtretenden Fluids enthalten.

Gleichfalls ist es möglich, die Hohlkanäle 17, 18 sowie den Durchgangskanal 9 mit einem Lichtleiter zu versehen. In diesem Fall bietet es sich an, die Komponente 24 als optische Einheit auszugestalten, bspw. in Form eines optischen Verstärkers, eines optischen Filters oder Schalters oder eines optischen Ein- und/oder Auskoppelelementes. Denkbar in diesem Zusammenhang ist eine optische Stimulation der intrakorporalen Bereiche A, B mit Licht, zusätzlich zu der ohnehin durch das Implantat realisierbaren elektrischen Stimulation. Innerhalb der implantierbaren Vorrichtung 2 gilt es hierzu wenigstens eine Lichtquelle vorzusehen, die optisch mit der optischen Einheit 24 gekoppelt ist. Ebenfalls kann innerhalb der implantierbaren Vorrichtung 2 ein Photodetektor enthalten sein, der intrakorporale optische Signale, die über den optischen Lichtleiter übertragbar sind, zu detektieren vermag.

Schließlich kann die Komponente 24 im Falle einer elektrischen Leitung längs der Hohlkanäle 17, 18 sowie des Durchgangskanal 9 in Form eines einstellbaren elektrischen Widerstandes oder eines elektrischen Verstärkers ausgebildet sein.

### Bezugszeichenliste

- 1: Kopfteil
- 2: implantierbare Vorrichtung, IPG
- 3: Dichtungsring
- 4: Kontaktringelement
- 51, 52: Steckerkontaktbuchse
- 61, 62: Öffnung
- 71, 72: Buchsenboden
- 8: Verbindungsmittel
- 9: Durchgangskanal
- 10: Innengewinde
- 12, 13: Steckeranordnung
- 14: N.N.
- 15: Gegenkontaktelement
- 16: Steckerkorpusbereich
- 17, 18: Hohlleitung
- 19: Dichtungs- und/oder Fügekontur
- 20, 21: abführende Leitung
- 23: Sensor
- 24: fluidische, optische oder elektrische Komponente

## Patentansprüche

1. Kopfteil (1) einer implantierbaren medizinischen Vorrichtung (2), mit einem Kopfteilgehäuse, das wenigstens eine erste sacklochartige Steckerkontaktbuchse (51) mit einer Buchsenöffnung (61) sowie einem der Buchsenöffnung (61) axial gegenüberliegenden Buchsenboden (71) besitzt, längs der in koaxialer Anordnung und axial serieller Abfolge wenigstens ein elektrisch leitendes Kontaktringelement (4) sowie ein elektrisch isolierender, elastisch verformbarer Dichtungsring (3) gefügt sind, die von einer erstarrten Vergussmasse umschlossen sind,
wobei wenigstens eine zweite sacklochartige Steckerkontaktbuchse (52) mit einer Buchsenöffnung (62) sowie einem der Buchsenöffnung (62) axial gegenüberliegenden Buchsenboden (72) innerhalb des Kopfteils (1) angeordnet ist, längs der in koaxialer Anordnung und axial serieller Abfolge wenigstens ein elektrisch leitendes Kontaktringelement (4) sowie ein elektrisch isolierender, elastisch verformbarer Dichtungsring (3) gefügt sind, die von der erstarrten Vergussmasse umschlossen sind, und
die Buchsenböden (71, 72) der wenigstens zwei Steckerkontaktbuchsen (51, 52) jeweils von einem von der erstarrten Vergussmasse zumindest teilweise umschlossenen Verbindungsmittel (8) begrenzt sind,
**dadurch gekennzeichnet, dass** das Verbindungsmittel (8) einen Durchgangskanal (9) einschliesst, der beidseitig in die sacklochartigen Steckerkontaktbuchsen (51, 52) offen mündet.

2. Kopfteil nach Anspruch 1,
**dadurch gekennzeichnet, dass** der Durchgangskanal (9) ein Hohlkanal ist.

3. Kopfteil nach Anspruch 1,
**dadurch gekennzeichnet, dass** der Durchgangskanal (9) mit einem Licht-leitenden Medium zumindest teilweise gefüllt ist.

4. Kopfteil nach Anspruch 1,
**dadurch gekennzeichnet, dass** der Durchgangskanal (9) mit einem elektrisch leitenden Medium gefüllt ist.

5. Kopfteil nach Anspruch 2,
**dadurch gekennzeichnet, dass** längs des Durchgangskanals (9) eine Fluidpumpe angeordnet ist.

6. Kopfteil nach Anspruch 3,
**dadurch gekennzeichnet, dass** längs des Durchgangskanals (9) eine optische Einheit (24) der nachfolgenden Art angeordnet ist: optischer Verstärker, optischer Filter oder Schalter, optisches Ein- und/oder Auskoppelelement.

7. Kopfteil nach Anspruch 4,
**dadurch gekennzeichnet, dass** längs des Durchgangskanals (9) eine elektrische Komponente (24) der nachfolgenden Art angeordnet ist: einstellbarer elektrischer Widerstand, elektrischer Verstärker.

8. Kopfteil nach einem der Ansprüche 1 bis 6,
**dadurch gekennzeichnet, dass** an das von der erstarrten Vergussmasse zumindest teilweise umschlossene Verbindungsmittels (8) wenigstens ein Sensor (23) angebracht ist zur sensorischen Erfassung eines innerhalb des Durchgangskanals (9) enthaltenden Mediums oder vorhandenen elektrischen Stromflusses.

9. Kopfteil nach einem der Ansprüche 1 bis 8,
**dadurch gekennzeichnet, dass** wenigstens ein Sensor (23) mit dem wenigstens einen elektrisch leitenden Kontaktringelement (4) elektrisch und/oder mechanisch verbunden ist.

10. Kopfteil nach Anspruch 8 oder 9,
**dadurch gekennzeichnet, dass** der wenigstens eine Sensor (23) aus der Gruppe der nachfolgenden Sensoren auswählbar ist: Ultraschallsensor, optischer Sensor, Drucksensor, Hall-Sensor, Flußsensor.

11. Kopfteil nach einem der Ansprüche 8 bis 10,
**dadurch gekennzeichnet, dass** der wenigstens eine Sensor (23) mit einer Auswerte- und Steuereinheit verbunden ist.

12. Kopfteil nach einem der Ansprüche 1 bis 11,
**dadurch gekennzeichnet, dass** das Verbindungsmittel (8) beidseitig, den Buchsenböden (71, 72) jeweils zugewandt, eine den Durchgangskanal (9) umfassende Fügekontur (19) aufweist, die derart ausgestaltet und angeordnet sind, so dass jeweils ein innerhalb der Steckerkontaktbuchsen (51, 52) angeordneter Medienkanal verlustfrei an den Durchgangskanal (9) ankoppelt.

13. Kopfteil nach einem der Ansprüche 1 bis 12,
**dadurch gekennzeichnet, dass** das Kopfteilgehäuse mechanisch und elektrisch mit einer als Pulsgenerator ausgebildeten implantierbaren, medizinischen Vorrichtung verbunden ist.

14. Kopfteil nach einem der Ansprüche 1 bis 13,
**dadurch gekennzeichnet, dass** die Längsachsen der ersten und zweiten sacklochartigen Steckerkontaktbuchse (51, 52) parallel zueinander orientiert sind, und deren Buchsenöffnungen (71, 72) an gegenüberliegenden Seiten oder an der gleichen Seite des Kopfteilgehäuse offen münden.

15. Verfahren zur Herstellung eines Kopfteils (1) nach einem der Ansprüche 1 bis 14,
wobei das den Durchgangskanal (9) umschließende Verbindungsmittel (8), das wenigstens eine Kontaktringelement (4) und der wenigstens eine Dichtungsring (3) längs der ersten sacklochartigen Steckerkontaktbuchse(51) längs eines ersten stabförmigen Montagewerkzeuges angeordnet und längs des ersten stabförmigen Montagewerkzeuges durch Erzeugen einer Fügekraft gegeneinander kraftschlüssig gefügt werden,
das den Durchgangskanal (9) umschließende Verbindungsmittel (8), das wenigstens eine Kontaktringelement (4) und der wenigstens eine Dichtungsring (3) längs der zweiten sacklochartigen Steckerkontaktbuchse (52) längs eines zweiten stabförmigen Montagewerkzeuges angeordnet und längs des zweiten stabförmigen Montagewerkzeuges durch Erzeugen einer Fügekraft gegeneinander kraftschlüssig gefügt werden, und
zumindest das längs des ersten und zweiten stabförmigen Montagewerkzeuges angeordnete und kraftschlüssig gefügte Verbindungsmittel (8), sowie das wenigstens eine Kontaktringelement (4) sowie der wenigstens eine Dichtungsring (3) der ersten und zweiten sacklochartigen Steckerkontaktbuchsen (51, 52) zumindest bereichsweise mit einer erstarrungsfähigen, in fließfähiger Form vorliegenden Vergußmasse ummantelt werden,
**dadurch gekennzeichnet, dass** das erste und zweite stabförmige Montagewerkzeug von dem Verbindungsmittel (8), dem wenigstens einen Kontaktringelement (4) sowie dem wenigstens einen Dichtungsring (3) der ersten und zweiten sacklochartigen Steckerkontaktbuchsen (51, 52) nach Erstarren der Vergußmasse, die in Form einer zumindest einen Teil des Kopfteilgehäuses bildenden, formstabilen Matrix die axiale Fügekraft mechanisch abstützt, derart gelöst und entfernt werden, so dass der das Verbindungsmittel (8) durchsetzende Durchgangskanal (9) beidseitig offen in die sich durch Entfernen der Montagewerkzeuge ausbildenden sacklochartigen Steckerkontaktbuchsen (51, 52) des Kopfteils (1) mündet.

## Claims

1. Head part (1) for an implantable medical device (2), with a head part housing that has at least one blind hole-like plug contact socket (51) with a socket opening (61) as well as a socket base (71) axially opposite the socket opening (61), fitted along which are, in coaxial arrangement and axially serial sequence, at least one electrically conducting contact ring element (4) as well as an electrically insulating, elastically deformable sealing ring (3), which are surrounded by a hardened casting compound,
wherein
arranged within the head part (1) is at least one second blind hole-like plug contact socket (52) with a socket opening (62) as well as a socket base (72) axially opposite the socket opening (62), fitted along which are, in coaxial arrangement and axially serial sequence, at least one electrically conducting contact ring element (4) as well as an electrically insulating, elastically deformable sealing ring (3), which are surrounded by a hardened casting compound,
in that the socket bases (71, 72) of the at least two plug contact sockets (51, 52) are delimited by a connection means (8) that is at least partially surrounded by the hardened casting compound, **characterised**
**in that** the connection means (8) encompasses a through channel (9), which bilaterally opens out into the blind hole-like plug contact sockets (51, 52).

2. Head part according to claim 1,
**characterised in that** the through channel (9) is a hollow channel.

3. Head part according to claim 1
**characterised in that** the through channel (9) is at least partially filled with a light-conducting medium.

4. Head part according to claim 1,
**characterised in that** the through channel (9) is filled with an electrically conductive medium.

5. Head part according to claim 2,
**characterised in that** a fluid pump is arranged along the through channel (9).

6. Head part according to claim 3,
**characterised in that** arranged along the through channel (9) there is an optical unit (24) of the following type: optical amplifier, optical filter or switch, optical coupling and/or decoupling element.

7. Head part according to claim 4,
**characterised in that** arranged along the through channel (9) is an electrical component (24) of the following type: adjustable electrical resistor, electrical amplifier.

8. Head part according to any one of claims 1 to 6,
**characterised in that** applied on the connection means (8) at least partially surrounded by the hardened casting compound, is at least one sensor (23) for the sensory detection of a medium contained within the through channel (9) or a present electric current flow.

9. Head part according to any one of claims 1 to 8
**characterised in that** the at least one sensor (23) is electrically and/or mechanically connected to an electrically conducting contact ring element (4).

10. Head part according to claim 8 or 9,
**characterised in that** the at least one sensor (23) can be selected from the group of the following sensors:
ultrasonic sensor, optical sensor, pressure sensor,
hall effect sensor, flow sensor.

11. Head part according to any one of claims 8 to 10
**characterised in that** the at least one sensor (23) is connected to an evaluation and control unit.

12. Head part according to any one of claims 1 to 11,
**characterised in that** on both sides the connection means (8) comprises a joining contour (19) surrounding the through channel (9) and in each case facing the socket bases (71, 72), that are configured and arranged so that in each case a media channel arranged within the plug contact socket (51, 52) connects to the through channel (9) in a leak-free manner.

13. Head part according to any one of claims 1 to 12,
**characterised in that** the head part housing is mechanically and electrically connected to an implantable medical device designed as a pulse generator.

14. Head part according to any one of claims 1 to 13,
**characterised in that** the longitudinal axes of the first and the second blind hole-like plug contact socket (51. 52) are orientated in parallel to each other and their socket openings (71, 72) open out on opposite sides or on the same side of the head part housing.

15. Method for producing a head part (1) according to any one of claims 1 to 14.
wherein
that the connection means (8), the at least one contact ring element (4) and that at least one sealing ring (3) are arranged along the first blind hole-like plug contact socket (51) along a first rod-like assembly tool and fitted against each other in a force-fitting manner along the first rod-like assembly tool through the generation of a joining force,
that the connection means (8) encompassing the through channel (9), the at least one contact ring element (4) and the at least one sealing ring (3) are arranged along the second blind hole-like plug contact socket (52) along a second rod-like assembly too and fitted against each other in a force-fitting manner along the second rod-like assembly tool through the generation of a joining force,
that the connection means (8) as well as the at least one contact ring element (4) as well as the at least one sealing ring (3) of the first and second blind hole-like plug contact sockets (51, 52) arranged and fitted in a force-fitting manner along the first and second rod-like assembly tool, are surrounded, at least in sections, by a hardenable casting compound that is present in flowable form,
**characterised in that** the first and second rod-like assembly tool are detached and removed from the connection means (8), the at least one contact ring element (4) as well as the at least one sealing ring (3) of the first and second blind hole-like plug contact socket (51, 52) after hardening of the casting compound, which mechanically supports the axial joining force in the form of a dimensionally stable matrix forming at least one part of the head part housing, in such a way that the through channel (9) passing through the connection means (8) bilaterally opens out into the blind hole-like plug contact sockets (51, 52) of the head part (1) formed through removing the assembly tools.

## Revendications

1. Partie de tête (1) d'un dispositive médical implantable (2) avec un boîtier de partie de tête, qui possède au moins une première douille de contact de connecteur de type trou borgne (51) avec une ouverture de douille (61) ainsi qu'un fond de douille (71) axialement opposé à l'ouverture de douille (61), le long de laquelle sont montés au moins un élément annulaire de contact électroconducteur (4) et une bague d'étanchéité (3) élastiquement déformable, électriquement isolante en montage coaxial et en succession axiale de série, qui sont entourés d'une masse de remplissage solidifiée, sachant qu'au moins une deuxième douille de contact de connecteur (52) de type trou borgne avec une ouverture de douille (62) et un fond de douille (72) axialement opposé à l'ouverture de douille (62) est disposée à l'intérieur de la partie de tête (1), le long de laquelle sont montés au moins un élément annulaire de contact électroconducteur (4) et une bague d'étanchéité (3) élastiquement déformable, électriquement isolante en montage coaxial et en succession axiale de série, qui sont entourés d'une masse de remplissage solidifiée, sachant que les fonds de douille (71, 72) d'au moins deux douilles de contact de connecteur (51, 52) sont respectivement limités par un moyen de liaison (8) entouré au moins en partie par une masse de remplissage solidifiée,
**caractérisée en ce que**
le moyen de liaison (8) comprend un conduit de passage (9), qui débouche ouvert des deux côtés dans les douilles de contact de connecteur de type trou borgne (51, 52).

2. Partie de tête selon la revendication 1,
**caractérisée en ce que**
le conduit de passage (9) est un conduit creux.

3. Partie de tête selon la revendication 1,
**caractérisée en ce que**
le conduit de passage (9) est rempli au moins en partie d'un milieu conducteur de lumière.

4. Partie de tête selon la revendication 1,
**caractérisée en ce que**
le conduit de passage (9) est rempli d'un milieu électroconducteur.

5. Partie de tête selon la revendication 2,
**caractérisée en ce qu'**
une pompe à fluide est disposée le long du conduit de passage (9).

6. Partie de tête selon la revendication 3,
**caractérisée en ce qu'**
une unité optique (24) du type ci-après est disposé le long du conduit de passage (9): amplificateur optique, filtre ou connecteur optique, élément de couplage et/ou découplage optique.

7. Partie de tête selon la revendication 4,
**caractérisée en ce qu'**
un composant électrique (24) du type ci-après est disposé le long du conduit de passage (9): résistance électrique réglable, amplificateur électrique.

8. Partie de tête selon l'une quelconque des revendications 1 à 6,
**caractérisée en ce qu'**au moins un capteur (23) est placé sur le moyen de liaison (8) au moins en partie entouré par la masse de remplissage solidifiée pour la saisie de détection d'un milieu contenu à l'intérieur du conduit de passage (9) ou d'un flux de courant électrique existant.

9. Partie de tête selon l'une quelconque des revendications 1 à 8,
**caractérisée en ce qu**'au moins un capteur (23) est relié électriquement et/ou mécaniquement à au moins un élément annulaire de contact (4) électroconducteur.

10. Partie de tête selon la revendication 8 ou 9,
**caractérisée en ce qu**'au moins un capteur (23) peut être choisi à partir du groupe des capteurs suivants : capteurs à ultrasons, capteurs optiques, capteurs de pression, capteurs de Hall, capteurs de débit.

11. Partie de tête selon l'une quelconque des revendication 8 à 10,
**caractérisée en ce qu**'au moins un capteur (23) est relié à une unite d'évaluation et de commande.

12. Partie de tête selon l'une quelconque des revendications 1 à 11,
**caractérisée en ce que** le moyen de liaison (8) comporte des deux côtés un contour de jonction (19) comprenant le conduit de passage (9), respectivement tourné vers les fonds de douille (71, 72), qui est structuré et disposé de telle manière qu'un conduit de milieu dispose respectivement à l'intérieur des douilles de contact de connecteur (51, 52) est accouplé sans perte au conduit de passage (9).

13. Partie de tête selon l'une quelconque des revendications 1 à 12,
**caractérisée en ce que** le boîtier de partie de tête est relié mécaniquement et électriquement à un dispositive medical implantable constitué sous la forme d'un générateur d'impulsions.

14. Partie de tête selon l'une quelconque des revendications 1 à 13,
**caractérisée en ce que** les axes longitudinaux de la première et de la deuxième douille de contact de connecteur de type trou borgne (51, 52) sont disposés parallèlement l'un à l'autre et dont les ouvertures de douille (71, 72) débouchent ouvertes sur les côtés opposés ou sur le même coté du boîtier de partie de tête.

15. Procédé de fabrication d'une partie de tête (1) selon l'une quelconque des revendications 1 à 14,
sachant que le moyen de liaison (8) entourant le conduit de passage (9), qui comporte au moins un élément annulaire de contact (4) et qui comporte au moins une bague d'étanchéité (3) le long de la première douille de contact de type trou borgne (51) disposé le long d'un premier outil de montage en forme de barre et sont assemblés l'un contre l'autre par conformité de force le long du premier outil de montage en forme de barre en produisant une force d'assemblage ,
sachant que le moyen de liaison (8) entourant le conduit de passage (9), qui comporte au moins un élément annulaire de contact (4) et qui comporte au moins une bague d'étanchéité (3) le long de la deuxième douille de contact de type trou borgne (52) disposé le long d'un deuxième outil de montage en forme de barre et sont assemblés l'un contre l'autre par conformité de force le long du deuxième outil de montage en forme de barre en produisant une force d'assemblage,
sachant qu'au moins le moyen de liaison (8) disposé le long du premier et du deuxième outil de montage en forme de barre et assemble par conformité de force ainsi qu'au moins une élément annulaire de contact (4) et au moins une bague d'étanchéité (3) de la première et de la deuxième douille de contact de connecteur de type trou borgne (51, 52) sont enveloppées au moins par endroits par une masse de remplissage solidifiable, présente sous une forme fluide,
**caractérisé en ce que**
le premier et le deuxième outil de montage en forme de barre sont séparés et enlevés du moyen de liaison (8), d'au moins un élément annulaire de contact (4) et d'au moins une bague d'étanchéité (3) de la première et de la deuxième douille de contact de connecteur de type trou borgne (51, 52) après solidification de la masse de remplissage, qui assiste mécaniquement la force d'assemblage axiale sous la forme d'une matrice de forme stable formant au moins une partie du boîtier de partie de tête de telle manière que le conduit de passage (9) traversant le moyen de liaison (8) ouvert des deux côtés débouche dans les douilles de contact de connecteur de type trou borgne (51, 52) de la partie de tête (1) se constituent par enlèvement des outils de montage.
